# EUROPÄISCHE PATENTSCHRIFT

(11) **EP 1 794 121 B1**
(45) Veröffentlichungstag und Bekanntmachung des Hinweises auf die Patenterteilung: **25.12.2013**
(21) Anmeldenummer: 05782871.7
(22) Anmeldetag: 08.09.2005
(51) Int. Cl.: C07D 209/42, A61K 31/496

(54) **NEUARTIGE KRISTALLFORM VON (3-CYAN-1H-INDOL-7-YL)-[4-(4-FLUORPHENETHYL)-PIPERAZIN-1-YL]-METHANON, HYDROCHLORID**
NOVEL CRYSTALLINE FORM OF (3-CYANO-1H-INDOL-7-YL)-[4-(4-FLUOROPHENETHYL)-PIPERAZINE-1-YL]-METHANONE HYDROCHLORIDE
NOUVELLE FORME CRISTALLINE DE (3-CYAN-1H-INDOL-7-YL)-[4-(4-FLUORPHENETHYL)-PIPERAZINE-1-YL]-METHANONE CHLORHYDRATE

(30) Priorität: 28.09.2004 DE 102004047517
(43) Veröffentlichungstag der Anmeldung: 13.06.2007
(73) Patentinhaber: Merck Patent GmbH, 64293 Darmstadt (DE)
(72) Erfinder: BATHE, Andreas, 64283 Darmstadt (DE); HELFERT, Bernd, 64372 Ober-Ramstadt (DE); KNIERIEME, Ralf, 64846 Gross-Zimmern (DE); SAAL, Christoph, 64853 Otzberg (DE); KEINER, Ronald, 64285 Darmstadt (DE)
(86) Internationale Anmeldenummer: PCT/EP2005/009647
(87) Internationale Veröffentlichungsnummer: WO 2006/034774

(56) Entgegenhaltungen:
- WO-A-01/07435
- WO-A-02/059092
- WO-A-03/045392
- WO-A-2004/032932
- ADAMEC R ET AL: "Prophylactic and therapeutic effects of acute systemic injections of EMD 281014, a selective serotonin 2A receptor antagonist on anxiety induced by predator stress in rats" EUROPEAN JOURNAL OF PHARMACOLOGY, AMSTERDAM, NL, Bd. 504, Nr. 1-2, 3. November 2004 (2004-11-03), Seiten 79-96, XP004613494 ISSN: 0014-2999

## Beschreibung

Die vorliegende Erfindung betrifft eine bisher unbekannte Kristallform B von (3-Cyan-1H-indol-7-yl)-[4-(4-fluorphenethyl)-piperazin-1-yl]-methanon, Hydrochlorid (im folgenden als EMD281014 bezeichnet), Verfahren zur ihrer Herstellung sowie deren Verwendung zur Herstellung eines Arzneimittels.

### Hintergrund der Erfindung

Die Verbindung EMD281014 ist aus dem Europäischen Patent EP 1 198 453 B1 bekannt und hat folgende Struktur:

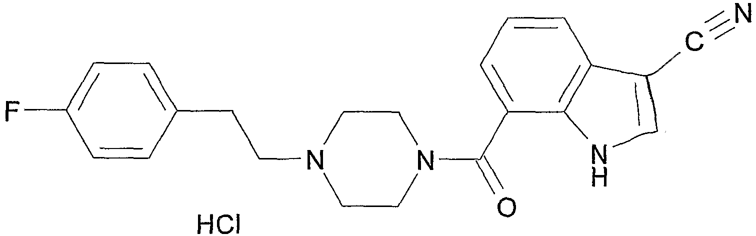

EMD281014 zeigt bei guter Verträglichkeit unter anderem Wirkungen auf das Zentralnervensystem und verfügt dabei über wertvolle pharmakologische Eigenschaften. So weist die Substanz eine starke Affinität zu 5-HT_{2A}-Rezeptoren auf, wobei sie 5-HT_{2A}-Rezeptor-antagonistische Eigenschaften besitzt.

Eine Reihe von medizinischen Verwendungen von EMD281014, bspw. die Behandlung von Schizophrenie und Schlafstörungen, sind in EP 1 198 453 B1 beschrieben. Weitere medizinische Verwendungen sind Gegenstand der WO 03/45392 und der WO 04/32932.

Verfahren zur Herstellung von EMD281014 sind in den Europäischen Patenten 1 198 453 B1 und 1 353 906 B1 offenbart.
Als abschließender Verfahrensschritt wird jeweils das Hydrochlorid aus einer Lösung der freien Base durch Versetzen mit einer wässrigen HCl-Lösung ausgefällt und aus dem Reaktionsgemisch abgetrennt.

Nach dieser bekannten Vorgehensweise wird stets eine Kristallform A erhalten, die durch die in Tabelle I angegebenen, durch Röntgen-Pulverdiffraktion bestimmten Netzebenenabstände gekennzeichnet ist.

Überraschenderweise wurde von den Erfindern der vorliegenden Patentanmeldung gefunden, daß sich beim Verpressen von EMD281014 zu Tabletten unter mechanischem Druck eine zweite Kristallform B bildet, die in signifikanten Mengen neben der Form A in den fertiggestellten Tabletten vorliegt. Die gebildete Menge an Form B hängt dabei vom angewendeten Pressdruck ab.

Nun ist es ausgesprochen unvorteilhaft, in einer Arzneimitteltablette mehrere Kristallformen eines Wirkstoffs nebeneinander vorliegen zu haben, wenn diese Kristallformen unterschiedliche Bioverfügbarkeiten aufweisen, indem sie bspw. unter physiologischen Bedinungen unterschiedlich schnell in Lösung gehen. Selbst bei geringfügigen Schwankungen der Herstellbedingungen wäre dann die Reproduzierbarkeit der Bioverfügbarkeit in Frage gestellt.

Aufgabe der vorliegenden Erfindung war es daher, EMD281014 in einer Form bereitzustellen, die unter den Bedingungen der Tablettierung ihre Eigenschaften nicht verändert und daher für die Herstellung von Tabletten mit definierter und gleichbleibender Qualität geeignet ist.

### Beschreibung der Erfindung

Überraschenderweise wurde gefunden, daß EMD281014 als Feststoff in unterschiedlichen Kristallmodifikationen vorliegen kann. Weiter wurde gefunden, daß sich der Kristallisationsprozess und damit die bevorzugte Entstehung einer der beiden Formen A oder B durch geeignete Wahl der Verfahrensparameter steuern läßt. Auch ist es möglich, Form A in Form B und Form B in Form A zu überführen, ohne EMD281014 dafür in Lösung bringen zu müssen.
Form B ist als unter den Bedingungen der Tablettenherstellung stabil anzusehen. Es ist nicht möglich, in den Röntgendiffraktogrammen von Tabletten, die aus der Form B hergestellt wurden, eindeutig Anteile der Form A oder von weiteren polymorphen Formen von EMD281014 zu identifizieren.

Beide Kristallformen enthalten ausschließlich EMD281014, also weder Wasser noch sonstige Solvensmoleküle.

Wie bereits erwähnt, wird nach den aus dem Stand der Technik bekannten Herstellverfahren Form A erhalten. Form A ist durch Röntgendaten gemäß Tabelle I gekennzeichnet.

**Tabelle I: Reflexlagen von EMD281014 Form A**

| **Nr.** | **d [Å]** | **Fehlerbreite d [Å]** | **I/I₀** |
|---|---|---|---|
| 1 | 14,132 | ± 0,25 | 53 |
| 2 | 8,939 | ± 0,10 | 17 |
| 3 | 6,304 | ± 0,05 | 19 |
| 4 | 6,013 | ± 0,05 | 85 |
| 5 | 5,388 | ± 0,05 | 60 |
| 6 | 5,293 | ± 0,05 | 37 |
| 7 | 5,193 | ± 0,05 | 27 |
| 8 | 4,927 | ± 0,05 | 52 |
| 9 | 4,369 | ± 0,05 | 18 |
| 10 | 4,224 | ± 0,05 | 63 |
| 11 | 4,167 | ± 0,02 | 50 |
| 12 | 4,078 | ± 0,02 | 23 |
| 13 | 3,812 | ± 0,02 | 79 |
| 14 | 3,691 | ± 0,02 | 41 |
| 15 | 3,434 | ± 0,02 | 71 |
| 16 | 3,383 | ± 0,02 | 100 |
| 17 | 3,330 | ± 0,02 | 17 |
| 18 | 3,207 | ± 0,02 | 19 |
| 19 | 3,134 | ± 0,02 | 23 |
| 20 | 3,027 | ± 0,02 | 38 |

Messbedingungen: Transmissionsbetrieb, Generatorleistung 40 kV/30 mA, Cu-Kα1-Strahlung (λ = 1,54056 Å), Ortsempfindlicher Detektor (3.3 kV), Meßbereich: 3-65 °2θ, Schrittgröße: 0,05 °2θ, Zeit/Schritt: 1,4 s
Auswertung: Die Diffraktogramme wurden im gesamten Aufnahmebereich 3-65 °2θ untergrundkorrigiert und die Reflexintensitäten der jeweils 20 stärksten Reflexe ermittelt. Die Toleranz der Winkellagen liegt bei ± 0,1 °2θ für die verwendete Cu-Kα1-Strahlung.

Um Form B in hoher Ausbeute und im wesentlichen rein herzustellen, wird folgendermaßen vorgegangen:
Zunächst wird die freie Base des EMD281014 in an sich bekannter Weise hergestellt und anschließend thermisch getrocknet, um anhaftende Solventien zu entfernen. Anstatt nun das Hydrochlorid durch Zugabe einer wässrigen HCl-Lösung zu fällen, wird HCl-Gas durch eine Lösung der freien Base geleitet. Hierbei wird ebenfalls ein Niederschlag erhalten, der jedoch überraschenderweise nicht aus der Form A sondern aus B besteht. Unter "Form B, im wesentlichen rein" oder "im wesentlichen bestehend aus Form B" wird hier verstanden, daß Form B weniger als 5%, vorzugsweise weniger als 2% und ganz bevorzugt weniger als 1 % Form A enthält.

Form B ist durch Röntgendaten gemäß Tabelle II gekennzeichnet.

**Tabelle II: Reflexlagen von EMD281014 Form B**

| **No.** | **d [Å]** | **Fehlerbreite d [Å]** | **I/I₀** |
|---|---|---|---|
| 1 | 13.083 | ± 0.20 | 30 |
| 2 | 6.688 | ± 0.10 | 77 |
| 3 | 5.669 | ± 0.05 | 55 |
| 4 | 5.292 | ± 0.05 | 100 |
| 5 | 4.786 | ± 0.05 | 41 |
| 6 | 4.040 | ± 0.02 | 46 |
| 7 | 3.881 | ± 0.02 | 28 |
| 8 | 3.514 | ± 0.02 | 37 |
| 9 | 3.239 | ± 0.02 | 28 |
| 10 | 3.200 | ± 0.02 | 25 |

Messbedingungen und Auswertung erfolgen wie bei Tabelle I beschrieben.

In einer bevorzugten Ausführungsform ist die Form B durch Röntgendaten gemäß Tabelle IIa gekennzeichnet. Die Daten gemäß Tabelle IIa enthalten die Reflexe aus Tabelle II und zusätzlich 10 weitere Reflexe geringerer Intensität.

**Tabelle IIa: Reflexlagen von EMD281014 Form B**

| **Nr.** | **d [Å]** | **Fehlerbreite d [Å]** | **I/I₀** |
|---|---|---|---|
| 1 | 13,083 | ± 0,20 | 30 |
| 2 | 8,706 | ± 0,10 | 19 |
| 3 | 6,688 | ± 0,10 | 77 |
| 4 | 6,499 | ± 0,05 | 19 |
| 5 | 5,669 | ± 0,05 | 55 |
| 6 | 5,292 | ± 0,05 | 100 |
| 7 | 4,786 | ± 0,05 | 41 |
| 8 | 4,322 | ± 0,05 | 23 |
| 9 | 4,040 | ± 0,02 | 46 |
| 10 | 3,881 | ± 0,02 | 28 |
| 11 | 3,595 | ± 0,02 | 14 |
| 12 | 3,514 | ± 0,02 | 37 |
| 13 | 3,435 | ± 0,02 | 22 |
| 14 | 3,337 | ± 0,02 | 14 |
| 15 | 3,289 | ± 0,02 | 25 |
| 16 | 3,239 | ± 0,02 | 28 |
| 17 | 3,200 | ± 0,02 | 25 |
| 18 | 3,143 | ± 0,02 | 18 |
| 19 | 3,073 | ± 0,02 | 22 |
| 20 | 2,867 | ± 0,01 | 19 |

Messbedingungen und Auswertung erfolgen wie bei Tabelle I beschrieben.

Gegenstand der vorliegenden Erfindung ist demnach eine polymorphe Kristallform B von EMD281014, gekennzeichnet durch die in Tabelle II angegebenen charakteristischen Gitterebenenabstände.
Insbesondere ist Gegenstand der vorliegenden Erfindung eine polymorphe Kristallform B von EMD281014, gekennzeichnet durch die in Tabelle IIa angegebenen charakteristischen Gitterebenenabstände.

Gegenstand ist weiterhin ein Verfahren zur Herstellung der Kristallform B von EMD281014 aus einer Lösung der freien Base von EMD281014 dadurch gekennzeichnet, daß durch diese Lösung HCl-Gas geleitet wird und der sich bildende Niederschlag abgetrennt wird.
In der Regel wird hierbei mit molaren Überschüssen von Lösemittel zu gelöstem Stoff von 50:1 bis 200:1 gearbeitet. Vorzugsweise jedoch 100:1 bis 150:1. Ein bevorzugtes Lösemittel ist Tetrahydrofuran (THF).
Es wurde weiterhin gefunden, daß Form B auch durch Rühren einer Suspension von Kristallen der Form A in tert.-Butylmethylether (MTBE) erhalten werden kann.
Ein entsprechendes Herstellverfahren ist daher ebenfalls Gegenstand der vorliegenden Erfindung.
Führt man das Verfahren bei Raumtemperatur 14 Tage lang durch, werden etwa 30% Form B neben Form A erhalten, wobei das Verhältnis von B zu A durch Vergleich des Röntgenpulverdiffraktogramms des Gemischs mit den Diffraktog rammen der Reinsubstanzen abgeschätzt wird. Durch die Wahl kürzerer oder längerer Reaktionszeiten können Gemische von B und A in beliebigen Zusammensetzungen hergestellt werden. Gegenstand der vorliegenden Erfindung ist daher EMD281014, enthaltend die Form B. Erfindungsgemäß bevorzugte Gemische enthalten jeweils mehr als 10%, 20%, 30%, 40%, 50%, 60%, 70%, 80% bzw. 90% der Form B. Gegenstand ist weiterhin EMD281014, welches im wesentlichen aus der Form B besteht.

Ebenso ist es möglich, Form A durch Rühren einer Suspension von Kristallen der Form B herzustellen. In diesem Fall werden polare Lösungsmittel wie z.B. Aceton, Wasser oder Mischungen dieser beider Solventien eingesetzt. Ein bevorzugtes Gemisch ist hierbei Aceton/Wasser in einem Verhältnis von 55:45 Gew.%. Verwendet man Wasser als alleiniges Solvens, wird vorzugsweise bei saurem pH (insbesondere pH 1) gearbeitet. Ein entsprechendes Herstellverfahren ist daher ebenfalls Gegenstand der vorliegenden Erfindung.

Wie bereits erwähnt entsteht Form B aus Form A unter Druckeinwirkung. Gegenstand der vorliegenden Erfindung ist daher weiterhin ein Verfahren zur Herstellung der Kristallform B von EMD281014 dadurch gekennzeichnet, daß auf Kristalle der Form A mechanischer Druck ausgeübt wird. Vorzugsweise handelt es sich hierbei um Drücke wie sie üblicherweise bei der Tablettenherstellung herrschen, wenn Stempelpreßkräfte zwischen ca. 2 bis 16 kN, insbesondere zwischen 6 und 16 kN, als Maximalpreßkräfte zur Anwendung kommen. Es wurde festgestellt, daß der Anteil von Form B bei zunehmendem Druck steigt. Bei einem Tablettierungsvorgang in einer Exzenterpresse mit einer Dauer (Kontakzeit) von 310 ms und einer Maximalpreßkraft von 16 kN wird ein Gemisch von ca. 25% Form B und 75% Form A erhalten. Bei einem Tablettierungsvorgang in einer Exzenterpresse mit einer Kontaktzeit von 250 ms und einer Maximalpreßkraft von 6 kN beträgt der Anteil ca. 20% B.

Es wurde weiter ein sehr einfacher Weg zur Herstellung von Form A aus Form B gefunden. Dazu muß Form B lediglich bei erhöhten Temperaturen zwischen ca. 75 und ca. 225 °C, vorzugsweise 90 und 160 °C und besonders bevorzugt zwischen 110 und 140 °C gelagert werden. In Abhängigkeit von der Temperatur und der Dauer der Lagerung können verschiedene Umwandlungsgrade erzielt werden. Je nach angestrebtem Umwandlungsgrad kann die Dauer der Lagerung bei gegebener Temperatur zwischen einigen Minuten und mehreren Tagen betragen. Um hohe Umwandlungsgrade zu erreichen, wird die Form A vorzugsweise mehrere Stunden bis Tage gelagert. Geeignete Lagerzeiten sind etwa 4, 8, 12, 16, 20, 24, 36 oder 48 Stunden. Bei 105 °C kann eine vollständige Umwandlung von B in A beispielsweise durch Lagern über einen Zeitraum von 24 Stunden erzielt werden.

Ein entsprechendes Herstellverfahren von A aus B ist ebenfalls Geg enstand der vorliegenden Erfindung.

Schließlich sind Gegenstand der vorliegenden Erfindung die Verwendung der Form B als Arzneimittel bzw. zur Herstellung von pharmazeutischen Zubereitungen und Arzneimitteln und diese Zubereitungen und Arzneimittel als solche.
Die besagte Verwendung erfolgt analog zu der bekannten Form A wie in EP 1 198 453 B1, WO 03/45392 und WO 04/32932 beschrieben.

### Beispiele

### 1. Herstellung der Form B von EMD281014 aus einer Lösung der freien Base

50 g der EMD281014 zugrunde liegenden Base (3-Cyan-1 H-indol-7-yl)-[4-(4-fluorphenethyl)-piperazin-1-yl]-methanon werden in einem 2 Liter Rundkolben in 1400 ml THF gelöst. Anschließend wird auf 5 °C abgekühlt. Nun wird unter Kühlung mit einm Eis/Ethanolbad innheralb von 4 Minuten 20 g HCl-Gas (entsprechend einem molaren Überschuß von 4,1 bezogen auf die eingesetzte Base) eingeleitet. Es bildet sich ein weißer Niederschlag. Nach beendeter Reaktion wird der Ansatz noch 60 Minuten bei 25 bis 27°C nachgerührt. Anschließend wird der Niederschlag bei dieser Temperatur über einen Büchner-Trichter abfiltriert und in einem Vakuumtrockenschrank bei 23°C 18 Stunden lang getrocknet.
Es werden 53,3 g weiße, feste Kristalle erhalten (Ausbeute: 97% d.Th.), die der Kristallform B entsprachen (Nachweis über Röntgenpulverdiffraktogramm).

Die so erhaltenen Kristalle weisen charakteristische Gitterebenenabstände auf, wie in Tabelle II und/oder IIa angegeben. Zur weiteren Charakterisierung wird ein Raman-Spektrum aufgenommen, welches die in Tabelle III aufgeführten typischen Banden zeigt.

**Tabelle III: Ramanbanden von EMD281014 Form B**

| **Wellenzahl [cm⁻¹]** | **Intensität** |
|---|---|
| 3075 ± 1,5 | M |
| 3066 ± 1,5 | M |
| 3057 ± 1,5 | M |
| 2994 ± 1,5 | M |
| 22961 ± 1,5 | M |
| 2927 ± 1,5 | M |
| 2219 ± 1,5 | S |
| 1629 ± 1,5 | M |
| 1611 ± 1,5 | M |
| 1604 ± 1,5 | W |
| 1594 ± 1,5 | W |
| 1525 ± 1,5 | M |
| 1447 ± 1,5 | M |
| 1342 ± 1,5 | M |
| 1333 ± 1,5 | M |
| 1298 ± 1,5 | M |
| 1250 ± 1,5 | M |
| 1160 ± 1,5 | M |
| 858 ± 1,5 | M |
| 826 ± 1,5 | M |
| 689 ± 1,5 | M |
| 637 ± 1,5 | M |
| 627 ± 1,5 | M |
| 503 ± 1,5 | M |

Messbedingungen: FT-Raman-Spektroskopie, Bruker RFS 100, 1064 nm Anregung, 750 mW, 1 cm-1 spektrale Auflösung, 250 Scans.
Auswertung: Das erhaltene Raman-Spektrum wird im Spektralbereich 3600 - 250 cm-1 vektornormiert. Die Banden werden aufgrund ihrer Intensität wie folgt in s = strong, m = middle und w = weak eingeteilt:

| | |
|---|---|
| S | I > 0,075 |
| M | 0,01 < I < 0,075 |
| W | I < 0,01 |

### 2. Vergleichsbeispiel: Herstellung der Form A von EMD281014 aus einer Lösung der freien Base wie in EP 1 353 906 B1 beschrieben

2,1 g der freien Base von EMD281014 werden in 50 ml Aceton erhitzt und bis zur klaren Lösung mit Wasser versetzt. Dann wird ein Gemisch aus 0,6 ml Salzsäure (w=37%) und 1,2 ml Aceton eingerührt. Anschließend wird am Rotationsverdampfer auf das halbe Volumen eingeengt. Das ausgefallene Hydrochlorid wird abgesaugt, mit Aceton und Diethylether gewaschen und getrocknet. Man erhält 1,6 g 7-{4-[2-(4-Fluor-phenyl)-ethyl]-piperazin-1-carbonyl}-1H-indol-3-carbonitril, Hydrochlorid (69 % der Theorie), Zersetzungsbereich 314 - 319°.

Die so erhaltenen Kristalle weisen charakteristische Gitterebenenabstände auf, wie in Tabelle II und/oder IIa angegeben. Zur weiteren Charakterisierung wird ein Raman-Spektrum aufgenommen, welches die in Tabelle IV aufgeführten typischen Banden zeigt.

**Tabelle IV: Ramanbanden von EMD281014 Form A**

| **Wellenzahl [cm⁻¹]** | **Intensität** |
|---|---|
| 3083 ± 1,5 | M |
| 3068 ± 1,5 | M |
| 3058 ± 1,5 | M |
| 3007 ± 1,5 | M |
| 2990 ± 1,5 | M |
| 2960 ± 1,5 | M |
| 2941 ± 1,5 | M |
| 2224 ± 1,5 | S |
| 1634 ± 1,5 | M |
| 1613 ± 1,5 | M |
| 1602 ± 1,5 | M |
| 1596 ± 1,5 | M |
| 1530 ± 1,5 | S |
| 1441 ± 1,5 | M |
| 1345 ± 1,5 | M |
| 1331 ± 1,5 | M |
| 1294 ± 1,5 | M |
| 1246 ± 1,5 | M |
| 1157 ± 1,5 | M |
| 859 ± 1,5 | M |
| 831 ± 1,5 | M |
| 824 ± 1,5 | M |
| 691 ± 1,5 | M |
| 638 ± 1,5 | M |
| 625 ± 1,5 | W |
| 505 ± 1,5 | W |
| 499 ± 1,5 | W |

Messbedingungen und Auswertung erfolgen wie bei Tabelle III beschrieben.

### 3. Herstellung von Form B von EMD281014 aus Form A durch Rühren einer Suspension von A in MTBE

250 mg EMD281014 Form A werden in 5 mL MTBE dispergiert und bei Raumtemperatur in einem verschlossenen Braunglas-Gefäß für 14 Tage gerührt: Der Rückstand wird über einen Papier-Rundfilter abfiltriert und an Raumluft getrocknet.
Ergebnis der Röntgenbeugungs-Messung: Es liegt ein Gemisch aus EMD281014 Form A und Form B vor. Der Anteil der Form B wird durch Vergleich der Röntgenpulverdiffraktogramme der reinen Formen auf ca. 30 Gew.% geschätzt.

### 4. Herstellung von Form B aus Form A durch Druckanwendung

In einer Exzenterpresse des Typs EKO der Firma Korsch (Berlin, Deutschland), Baujahr 2002, werden Tabletten bei einer Geschwindigkeit von 50 Stück pro Minute hergestellt, wobei Stempel 7 mm rund, flach mit Facette verwendet werden.
Die Tabletten enthalten 50 mg EMD281014 der Kristallform A, 93,2 mg Lactosemonohydrat, 4,5 mg Croscarmellose und 2,3 mg Magnesiumstearat. Die Ingredientien werden trocken gemischt und direkt komprimiert.

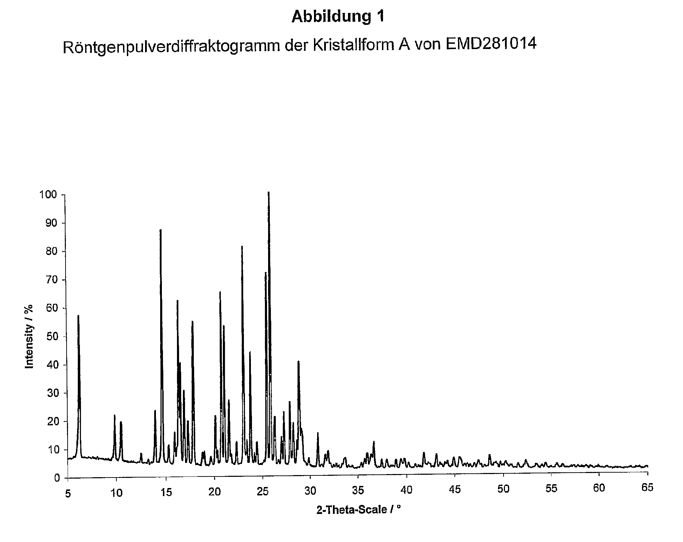

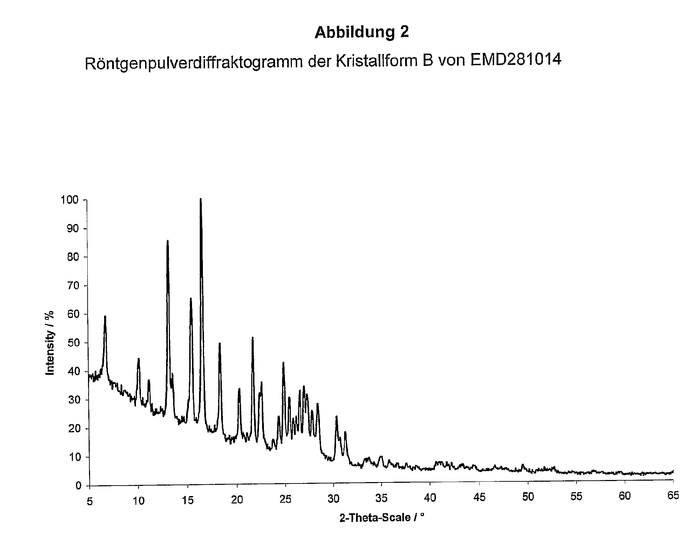

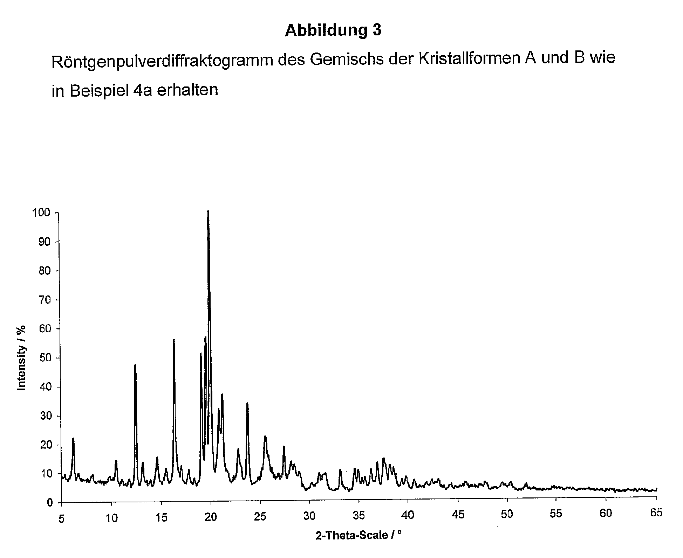

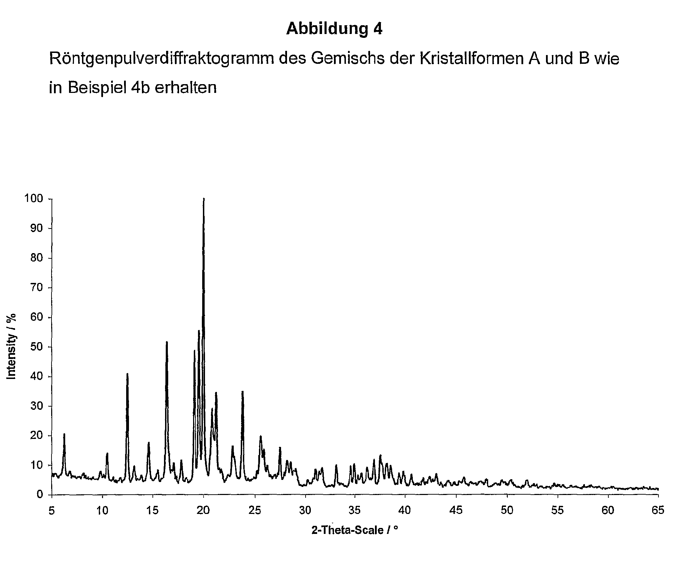

Der Anteil von Form B wird durch Vergleich der Röntgenpulverdiffraktogramme der Gemische (Abbildungen 3 und 4) mit den Diffraktogrammen der reinen Formen (Abbildungen 1 und 2) abgeschätzt.

Es werden folgende Ergebnisse erhalten:

| Beispiel | 4a | 4b |
|---|---|---|
| Mittlere maximale Oberstempelpreßkraft [kN] | 16 | 6 |
| Fläche unter Preßkraftkurve [kN ms] | 2780 | 800 |
| Kontaktzeit [ms] | 310 | 270 |
| Anteil Form B | 25 | 20 |

## Patentansprüche

1. Kristallform B von (3-Cyan-1 H-indol-7-yl)-[4-(4-fluorphenethyl)-piperazin-1-yl]-methanon, Hydrochlorid, **gekennzeichnet durch** folgende charakteristische Gitterebenenabstände in Å: 13.083 ± 0.20, 6.688 ± 0.10, 5.669 ± 0.05, 5.292 ± 0.05, 4.786 ± 0.05, 4.040 ± 0.02, 3.881 ± 0.02, 3.514 ± 0.02, 3.239 ± 0.02, 3.200 ± 0.02.

2. Kristallform B von (3-Cyan-1H-indol-7-yl)-[4-(4-fluorphenethyl)-piperazin-1-yl]-methanon, Hydrochlorid, gemäß Anspruch 1, **gekennzeichnet durch** folgende charakteristische Gitterebenenabstände in Å: 13,083 ± 0,20, 8,706 ± 0,10, 6,688 ± 0,10, 6,499 ± 0,05, 5,669 ± 0,05, 5,292 ± 0,05, 4,786 ± 0,05, 4,322 ± 0,05, 4,040 ± 0,02, 3,881 ± 0,02, 3,595 ± 0,02, 3,514 ± 0,02, 3,435 ± 0,02, 3,337 ± 0,02, 3,289 ± 0,02, 3,239 ± 0,02, 3,200 ± 0,02, 3,143 ± 0,02, 3,073 ± 0,02, 2,867 ± 0,01.

3. (3-Cyan-1H-indol-7-yl)-[4-(4-fluorphenethyl)-piperazin-1-yl]-methanon, Hydrochlorid, enthaltend die Kristallform B gemäß Anspruch 1 oder 2.

4. (3-Cyan-1H-indol-7-yl)-[4-(4-fluorphenethyl)-piperazin-1-yl]-methanon, Hydrochlorid, im wesentlichen bestehend aus Kristallform B gemäß Anspruch 1 oder 2.

5. Verfahren zur Herstellung der Kristallform B gemäß Anspruch 1 oder 2 aus der bekannten Form A, **gekennzeichnet durch** Ausüben von mechanischem Druck.

6. Verfahren zur Herstellung der Kristallform B gemäß Anspruch 1 oder 2, **dadurch gekennzeichnet, daß** durch eine Lösung von (3-Cyan-1 H-indol-7-yl)-[4-(4-fluorphenethyl)-piperazin-1-yl]-methanon HCl-Gas geleitet wird und der sich bildende Niederschlag aus dem Reaktionsgemisch abgetrennt und getrocknet wird.

7. Verfahren zur Herstellung der Kristallform B gemäß Anspruch 1 oder 2, **dadurch gekennzeichnet, daß** eine Suspension von Kristallen der bekannten Form A in tert.-Butylmethylether gerührt wird und der Rückstand aus dem Reaktionsgemisch abgetrennt und getrocknet wird.

8. Kristallform B von (3-Cyan-1 H-indol-7-yl)-[4-(4-fluorphenethyl)-piperazin-1-yl]-methanon, Hydrochlorid, gemäss Anspruch 1 oder 2, erhältlich nach einem Verfahren gemäß Anspruch 5, 6 oder 7.

9. Kristallform gemäß Anspruch 1, 2, 3, 4 oder 8 als Arzneimittel.

10. Pharmazeutische Zubereitung, enthaltend die Kristallform gemäß Anspruch 1, 2, 3, 4 oder 8 und gegebenenfalls weitere Wirk- und/oder Hilfsstoffe.

11. Verwendung der Kristallform gemäß Anspruch 1, 2, 3, 4 oder 8 zur Herstellung eines Arzneimittels.

12. Verfahren zur Herstellung der bekannten Kristallform A aus der Kristallform B gemäß Anspruch 1 oder 2, **dadurch gekennzeichnet, daß** die Form B bei einer erhöhten Temperatur von ca. 75 bis ca. 225 °C gelagert wird.

13. Verfahren zur Herstellung der bekannten Kristallform A aus der Kristallform B gemäß Anspruch 1 oder 2, **dadurch gekennzeichnet, daß** eine Suspension von Kristallen der Form B in einem polaren Lösemittel oder Lösemittelgemisch gerührt wird und der Rückstand aus dem Reaktionsgemisch abgetrennt und getrocknet wird.

## Claims

1. Crystal form B of (3-cyano-1H-indol-7-yl)-[4-(4-fluorophenethyl)-piperazin-1-yl]methanone, hydrochloride, **characterised by** the following characteristic interlattice plane distances in A: 13.083 ± 0.20, 6.688 ± 0.10, 5.669 ± 0.05, 5.292 ± 0.05, 4.786 ± 0.05, 4.040 ± 0.02, 3.881 ± 0.02, 3.514 ± 0.02, 3.239 ± 0.02, 3.200 ± 0.02.

2. Crystal form B of (3-cyano-1H-indol-7-yl)-[4-(4-fluorophenethyl)-piperazin-1-yl]methanone, hydrochloride, according to Claim 1, **characterised by** the following characteristic interlattice plane distances in Å: 13.083 ± 0.20, 8.706 ± 0.10, 6.688 ± 0.10, 6.499 ± 0.05, 5.669 ± 0.05, 5.292 ± 0.05, 4.786 ± 0.05, 4.322 ± 0.05, 4.040 ± 0.02, 3.881 ± 0.02, 3.595 ± 0.02, 3.514 ± 0.02, 3.435 ± 0.02, 3.337 ± 0.02, 3.289 ± 0.02, 3.239 ± 0.02, 3.200 ± 0.02, 3.143 ± 0.02, 3.073 ± 0.02, 2.867 ± 0.01.

3. (3-Cyano-1H-indol-7-yl)-[4-(4-fluorophenethyl)piperazin-1-yl]methanone, hydrochloride, comprising crystal form B according to Claim 1 or 2.

4. (3-Cyano-1H-indol-7-yl)-[4-(4-fluorophenethyl)piperazin-1-yl]methanone, hydrochloride, essentially consisting of crystal form B according to Claim 1 or 2.

5. Process for the preparation of crystal form B according to Claim 1 or 2 from the known form A, **characterised by** the exertion of mechanical pressure.

6. Process for the preparation of crystal form B according to Claim 1 or 2, **characterised in that** HCl gas is passed through a solution of (3-cyano-1H-indol-7-yl)-[4-(4-fluorophenethyl)piperazin-1-yl]methanone, and the precipitate which forms is separated off from the reaction mixture and dried.

7. Process for the preparation of crystal form B according to Claim 1 or 2, **characterised in that** a suspension of crystals of the known form A in tert-butyl methyl ether is stirred, and the residue is separated off from the reaction mixture and dried.

8. Crystal form B of (3-cyano-1 H-indol-7-yl)-[4-(4-fluorophenethyl)-piperazin-1-yl]methanone, hydrochloride, according to Claim 1 or 2, obtainable by a process according to Claim 5, 6 or 7.

9. Crystal form according to Claim 1, 2, 3, 4 or 8 as medicament.

10. Pharmaceutical composition comprising the crystal form according to Claim 1, 2, 3, 4 or 8 and optionally further active ingredients and/or adjuvants.

11. Use of the crystal form according to Claim 1, 2, 3, 4 or 8 for the preparation of a medicament.

12. Process for the preparation of the known crystal form A from crystal form B according to Claim 1 or 2, **characterised in that** form B is stored at an elevated temperature of from about 75 to about 225°C.

13. Process for the preparation of the known crystal form A from crystal form B according to Claim 1 or 2, **characterised in that** a suspension of crystals of form B in a polar solvent or solvent mixture is stirred, and the residue is separated off from the reaction mixture and dried.

## Revendications

1. Forme cristalline B de (3-cyano-1H-indol-7-yl)-[4-(4-fluorophénéthyl)-piperazin-1-yl]méthanone, hydrochlorure, **caractérisée par** les distances des plans inter-réseaux cristallins caractéristiques qui suivent en Å: 13,083 ± 0,20, 6,688 ± 0,10, 5,669 ± 0,05, 5,292 ± 0,05, 4,786 ± 0,05, 4,040 ± 0,02, 3,881 ± 0,02, 3,514 ± 0,02, 3,239 ± 0,02, 3,200 ± 0,02.

2. Forme cristalline B de (3-cyano-1H-indol-7-yl)-[4-(4-fluorophénéthyl)-piperazin-1-yl]méthanone, hydrochlorure, selon la revendication 1, **caractérisée par** les distances des plans inter-réseaux cristallins caractéristiques qui suivent en Å: 13,083 ± 0,20, 8,706 ± 0,10, 6,688 ± 0,10, 6,499 ± 0,05, 5,669 ± 0,05, 5,292 ± 0,05, 4,786 ± 0,05, 4,322 ± 0,05, 4,040 ± 0,02, 3,881 ± 0,02, 3,595 ± 0,02, 3,514 ± 0,02, 3,435 ± 0,02, 3,337 ± 0,02, 3,289 ± 0,02, 3,239 ± 0,02, 3,200 ± 0,02, 3,143 ± 0,02, 3,073 ± 0,02, 2,867 ± 0,01.

3. (3-Cyano-1H-indol-7-yl)-[4-(4-fluorophénéthyl)piperazin-1-yl]méthanone, hydrochlorure, comprenant la forme cristalline B selon la revendication 1 ou 2.

4. (3-Cyano-1H-indol-7-yl)-[4-(4-fluorophénéthyl)piperazin-1-yl]méthanone, hydrochlorure, constitué essentiellement par la forme cristalline B selon la revendication 1 ou 2.

5. Procédé pour la préparation de la forme cristalline B selon la revendication 1 ou 2 à partir de la forme connue A, **caractérisé par** l'exercice d'une pression mécanique.

6. Procédé pour la préparation de la forme cristalline B selon la revendication 1 ou 2, **caractérisé en ce que** du gaz HCl est passé au travers d'une solution de (3-cyano-1H-indol-7-yl)-[4-(4-fluorophénéthyl)piperazin-1-yl]méthanone, et le précipité qui se forme est séparé du mélange de réaction et est séché.

7. Procédé pour la préparation de la forme cristalline B selon la revendication 1 ou 2, **caractérisé en ce qu'**une suspension de cristaux de la forme connue A dans du méthyléther de tert-butyle est agitée, et le résidu est séparé du mélange de réaction et est séché.

8. Forme cristalline B de (3-cyano-1H-indol-7-yl)-[4-(4-fluorophénéthyl)-piperazin-1-yl]méthanone, hydrochlorure, selon la revendication 1 ou 2, pouvant être obtenue au moyen d'un procédé selon la revendication 5, 6 ou 7.

9. Forme cristalline selon la revendication 1, 2, 3, 4 ou 8 en tant que médicament.

10. Composition pharmaceutique comprenant la forme cristalline selon la revendication 1, 2, 3, 4 ou 8 et en option, d'autres ingrédients actifs et/ou adjuvants.

11. Utilisation de la forme cristalline selon la revendication 1, 2, 3, 4 ou 8 pour la préparation d'un médicament.

12. Procédé pour la préparation de la forme cristalline connue A à partir d'une forme cristalline B selon la revendication 1 ou 2, **caractérisé en ce que** la forme B est stockée à une température élevée qui va d'environ 75 à environ 225°C.

13. Procédé pour la préparation de la forme cristalline connue A à partir de la forme cristalline B selon la revendication 1 ou 2, **caractérisé en ce qu'**une suspension de cristaux de la forme B dans un solvant polaire ou un mélange de solvants polaires est agitée, et le résidu est séparé du mélange de réaction et est séché.
